# EUROPEAN PATENT APPLICATION

(11) **EP 3 308 755 A1**
(43) Date of publication of application: **18.04.2018**
(21) Application number: 17001435.1
(22) Date of filing: 25.08.2017
(51) Int. Cl.: A61F 9/00

(54) **OPHTHALMIC PROBES**

(30) Priority: 25.08.2016 GB 201614487
(71) Applicant: Blink Medical Limited, Shirley Solihull B90 4NS (GB)
(72) Inventor: Tyler, Roger, Shirley, Solihull B90 4NS (GB)
(74) Representative: Coles, Graham Frederick

(57) **Abstract**

An ophthalmic probe (11) for use in sub-tenon blocking has an elongate injection-moulded plastics stem (12) with an angled pointed conical tip (13) at one end for piercing and manipulation of the conjunctiva to admit entry of a cannula administrating liquid anaesthetic. Finger-hold pairs (14,15) at intermediate locations along the stem (12) enable controlled manipulation of the probe (11) during surgery, and an optional dissector (16) with blunt tip and edge (17) at the other end of the stem (12) may be used without cutting, in opening a pocket for the anaesthetic.

## Description

This invention relates to ophthalmic probes for use during ophthalmic surgical procedures, and is concerned, though not exclusively, with conjunctival probes for use in sub-tenon local anaesthesia.

In sub-tenon local anaesthesia, or as referred to alternatively as a "sub-tenon block" procedure, ophthalmic scissors are used to make an initial opening cut in the conjunctiva, and it is into the pocket under the sub-tenon capsule so formed, that local anaesthetic solution is administered via a blunt ophthalmic cannula inserted in the opening.

If the opening cut made in the conjunctiva is larger than required for entry of the ophthalmic cannula, there is the disadvantage that the local anaesthetic solution will to some extent escape and reduce the required sub-tenon anaesthetic block. However, significant skill in use of the scissors to make the cut is required, and there is difficulty with them in limiting the size of the resultant opening.

It is an object of the present invention to provide for reduction of the skill required in the procedure of making the opening in the conjunctiva and to reduce the difficulty of limiting the size of the opening made.

According to the present invention there is provided an ophthalmic probe having an elongate stem, and a pointed tip carried by the elongate stem for piercing a conjunctiva to make an opening in it.

The pointed tip of the ophthalmic probe of the invention may be located at an end of the elongate stem, and may be inclined to it, and may be conical.

The piercing of the conjunctiva with the pointed tip of the probe enables a controlled and limited opening to be made and may be facilitated by stretching or tenting the conjunctiva using a pair of forceps.

A dissector may be carried by the elongate stem for manipulation of the tissues of the conjunctiva pierced by the pointed tip. The dissector may be located at an opposite end of the elongate stem from the pointed tip, and may have a blunt tip and edge, and may be in the form of blade or plate.

Two forms of ophthalmic probe in accordance with the present invention will now be described, by way of example, with reference to the accompanying drawings in which:
Figure 1 is a longitudinal elevation of a first, single-ended ophthalmic probe in accordance with the invention; and
Figure 2 is a longitudinal elevation of a double-ended ophthalmic probe according to the invention.

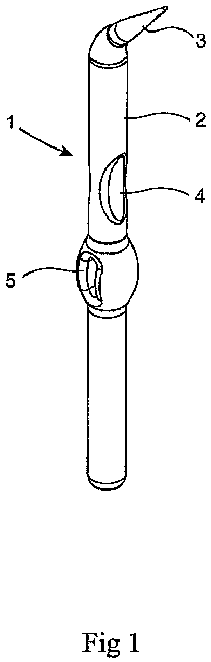

Referring to Figure 1, the single-ended ophthalmic probe 1 illustrated, has an elongate stem 2 with an angled pointed-tip 3 at one end. The tip 3, which is conical, is designed to make a small piercing with a controlled opening in the conjunctiva, and to enable the initial opening to be enlarged (for example by manipulation of the probe) to the extent necessary to achieve close entry of the blunt ophthalmic cannula for administration of the liquid sub-tenon anaesthetic.

The probe 1 is manufactured by injection moulding of a polycarbonate plastics material, and is intended for single use. Pairs of finger-grips 4 and 5 (only one of each pair is revealed in the drawing) are moulded indented into the stem 1 at intermediate locations along its length, to enable safe handling and tightly-controlled manipulation during surgical use.

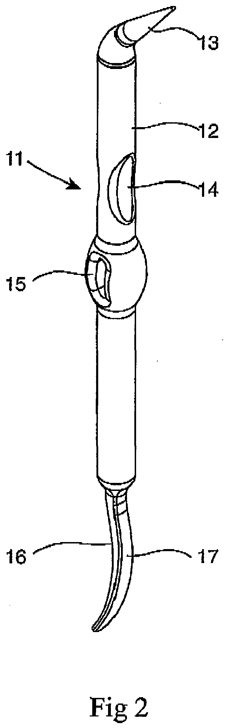

Although the probe 1 described with reference to Figure 1 is of a single-ended form, a double-ended form may also, or alternatively, be provided. In this respect, a double-ended probe 11 is illustrated in Figure 2, and will now be described.

Referring to Figure 2, the probe 11, like the probe 1, is manufactured by injection moulding of a polycarbonate plastics material, and is intended for single use. Additionally, it has an elongate stem 12 with an angled pointed-tip 13 corresponding to the tip 3 of the probe 1 at one end, and finger-grips 14 and 15 indenting the stem 12 at intermediate locations of its length. However, a dissector 16 with blunt tip and edge 17, is secured to the probe 11 at the opposite end of the stem 2 from the tip 13, to facilitate easy manipulation of the tissues beneath the conjunctiva when pierced by the tip 13. It can be used to open a pocket into which the liquid sub-tenon anaesthetic is injected from the ophthalmic cannula, and for strength broadens out from its tip and edge 17 which are blunt for use in gently separating, without cutting, any small areas binding the conjunctiva to the sclera.

## Claims

1. An ophthalmic probe having an elongate stem, and a pointed tip carried by the elongate stem for piercing a conjunctiva to make an opening in it.

2. An ophthalmic probe according to claim 1, wherein the pointed tip is located at an end of the elongate stem.

3. An ophthalmic probe according to claim 1 or claim 2, wherein the pointed tip is inclined at an angle to the elongate stem.

4. An ophthalmic probe according to any one of claims 1 Lo 3, wherein the pointed tip is conical.

5. An ophthalmic probe according to any one of claims 1 to 4, wherein a dissector is carried by the elongate stem for manipulation of the tissues of the conjunctiva pierced by the pointed tip.

6. An ophthalmic probe according to claim 5, wherein the dissector is located at an opposite end of the elongate stem from the pointed tip.

7. An ophthalmic probe according to claim 5 or claim 6, wherein the dissector has a blunt tip and edge.

8. An ophthalmic probe according to any one of claims 5 to 7, wherein the dissector is in the form of a blade or plate.

9. An ophthalmic probe according to any one of claims 1 to 8, wherein the elongate stem is of moulded plastics.

10. An ophthalmic probe according to any one of claims 1 to 9, wherein the elongate stem has finger-grips for use in manipulation of the probe during surgical use.
